# EUROPÄISCHE PATENTANMELDUNG

(11) **EP 3 616 659 A1**
(43) Veröffentlichungstag der Anmeldung: **04.03.2020**
(21) Anmeldenummer: 19193058.5
(22) Anmeldetag: 22.08.2019
(51) Int. Cl.: A61F 2/958

(54) **VERFAHREN UND VORRICHTUNG ZUM FIXIEREN EINES STENTS AUF EINEM BALLON EINES BALLONKATHETERS**

(30) Priorität: 28.08.2018 DE 102018120939
(71) Anmelder: BIOTRONIK AG, 8180 Bülach (CH)
(72) Erfinder: Fargahi, Amir, 8180 Bülach (CH)
(74) Vertreter: Galander, Marcus

(57) **Zusammenfassung**

Die Erfindung betrifft ein Verfahren zum Fixieren eines Implantats (1) auf einen Ballon (2), wobei ein erwärmter Ballon (2), der einen mit Druck beaufschlagbaren Balloninnenraum aufweist, mit einem auf den Ballon gecrimpten Implantat, das eine Stützstruktur (100) mit einer Vielzahl an Durchgangsöffnungen (101) aufweist, in einem Innenraum (7) einer Hülle (3) bereitgestellt wird, wobei im Innenraum (7) der Hülle (3) ein Unterdruck angelegt wird und der Balloninnenraum (6) mit einem Druck beaufschlagt wird, so dass der Ballon (2) in die Durchgangsöffnungen (101) des Implantats (1) tiefgezogen wird, wobei ein Formschluss zwischen dem Ballon (2) und dem Implantat (1) erzeugt wird. Weiterhin betrifft die Erfindung eine Vorrichtung (10) zur Durchführung des Verfahrens sowie eine durch das Verfahren hergestellte Anordnung (11).

## Beschreibung

Die Erfindung betrifft ein Verfahren und eine Vorrichtung zum Fixieren eines Implantats auf einem Ballon eines Ballonkatheters sowie eine Anordnung mit einem auf einem Ballon fixierten Implantat. Die vorliegende Erfindung wird am Beispiel eines Stents und eines Ballons eines Ballonkatheters beschrieben. Die vorliegende Erfindung ist jedoch prinzipiell zur Fixierung eines beliebigen Implantates auf einem Ballon geeignet. Derartige Implantate sind beispielsweise Stents, Herzklappenprothesen mit einem stentartigen Grundgerüst, Okkluder oder allgemein rohrförmige, ballonexpandierbare Implantate

Bei auf Ballons eines Ballonkatheter gecrimpten Implantaten, insbesondere Stents, ist es von hoher Wichtigkeit, dass die Implantate sicher auf dem jeweiligen Ballon eines Ballonkatheters fixiert sind, damit sie beim Implantieren nicht bezüglich des Ballons verrutschen. Es ist daher wichtig, eine ausreichende Implantat- oder Stenthaltekraft zu erzielen. Diese Stenthaltekraft stellt sich bei einen auf einen Ballon gecrimpten Implantat oder Stent als Summe aus Formschluss- und Kraftschlussverbindungen dar.

In den einschlägigen Normen (z. B. ASTM Norm F2394-07) wird - so auch im Rahmen der vorliegenden Erfindung - unter einem Crimpen eines Implantats/Stents auf einen Ballon ein Sichern des Stents auf dem Ballon verstanden, bei dem der Stent auf dem Ballon in radialer Richtung (zum gefalteten Ballon hin) komprimiert und dabei plastisch deformiert wird.

Im Hinblick auf die Stenthaltekraft, entstehen formschlüssige Verbindungen durch das Ineinandergreifen von mindestens zwei Verbindungspartnern (hier Stent und Ballon). Dadurch können sich die Verbindungspartner auch ohne oder bei unterbrochener Kraftübertragung nicht lösen. Anders ausgedrückt ist bei einer formschlüssigen Verbindung der eine Verbindungspartner dem anderen Verbindungspartner im Weg.

Kraftschlüssige Verbindungen setzen eine Normalkraft auf die miteinander zu verbindenden Flächen voraus. Ihre gegenseitige Verschiebung wird verhindert, solange die durch die Haftreibung bewirkte Gegenkraft nicht überschritten wird. Der Kraftbeziehungsweise Reibschluss ist verloren und die Flächen rutschen aufeinander, wenn die tangential wirkende Lastkraft größer als die Haftreibungskraft ist.

Hiervon ausgehend liegt der vorliegenden Erfindung die Aufgabe zugrunde, die Fixierung eines auf einen Ballon gecrimpten Implantats bezüglich des Ballons weiter zu verbessern, um das Risiko des Verrutschens des Implantats, insbesondere während einer Implantation, zu verhindern.

Diese Aufgabe wird durch ein Verfahren mit den Merkmalen des Anspruchs 1, eine Anordnung mit den Merkmalen des Anspruchs 12 sowie durch eine Vorrichtung mit den Merkmalen des Anspruchs 13 gelöst.

Ausführungsformen dieser Erfindungsaspekte sind in den entsprechenden Unteransprüchen angegeben und/oder werden nachfolgend beschrieben.

Gemäß Anspruch 1 wird ein Verfahren zum Fixieren eines Implantats auf einen Ballon (für einen Ballonkatheter) offenbart, wobei ein, bevorzugt erwärmter, Ballon, auf den ein Implantat gecrimpt ist, das eine Stützstruktur mit einer Vielzahl an Durchgangsöffnungen aufweist, in einem evakuierbaren Innenraum einer Hülle bereitgestellt wird, wobei im Innenraum der Hülle ein Unterdruck angelegt wird und der Balloninnenraum mit einem Druck beaufschlagt wird, so dass der, bevorzugt erwärmte, Ballon in die Durchgangsöffnungen des Implantats tiefgezogen wird, wobei ein Formschluss zwischen dem Ballon und dem Implantat bzw. der Stützstruktur des Implantats erzeugt wird. Das Bereitstellen des erwärmten Ballons im Innenraum der Hülle kann z. B. erfolgen, indem der Ballon außerhalb des Innenraumes der Hülle erwärmt wird (z. B. mittels einer geeigneten Heizvorrichtung) und sodann der erwärmte Ballon im Innenraum der Hülle angeordnet wird, oder indem der Ballon im Innenraum der Hülle angeordnet wird und im Innenraum der Hülle erwärmt wird (z. B. mittels einer in die Hülle integrierten oder mit der Hülle in thermischer Verbindung stehenden Heizvorrichtung).

Das erfindungsgemäße Verfahren eignet sich besonders zum Fixieren eines Stents auf einem Ballon eines Ballonkatheters. Unter Stent wird dabei im Rahmen dieser Anmeldung eine dauerhafte oder degradierbare rohrförmige Struktur verstanden, die in ein Körpergefäß, insbesondere ein Blutgefäß, implantiert werden kann. Der Stent kann dabei auch ein oder mehrere, insbesondere wirkstoffhaltige, Beschichtungen aufweisen. Derartige Stent weisen vorteilhafterweise eine Vielzahl von Streben auf, die die Struktur des Stents bilden, wobei die Streben zumeist mäanderförmig verlaufen und als Vielzahl von Ringen und/oder Helizes angeordnet und derart miteinander verbunden sind, dass sie eine im Wesentlichen zylindrische Struktur mit einer Vielzahl von Zwischenräumen bzw. Durchgangsöffnungen zwischen den Streben bilden.

Bei dem Verfahren wird also insbesondere im Innenraum der Hülle ein Unterdruck angelegt, der Balloninnenraum mit einem Druck beaufschlagt und der Ballon solchermaßen erwärmt (insbesondere vorher oder im Innenraum der Hülle), dass der Ballon eine Vielzahl an plastisch verformten Materialbereichen ausbildet, die jeweils formschlüssig in eine zugeordnete Durchgangsöffnung der Implantatstruktur hineinragen bzw. eingreifen, so dass der Formschluss zwischen dem Ballon und dem Implantat erzeugt wird.

Dieses erfindungsgemäße Unterdruck- bzw. Vakuumtiefziehen erzeugt somit einen hohen Umformgrad und damit eine optimale Formschlussverbindung zwischen dem Implantat und dem Ballon. Das führt mit Vorteil zu einer erhöhten Implantathaltekraft.

Die Durchgangsöffnungen des Implantats oder Stents werden auch als Zellen des Implantats bezeichnet. Die jeweilige Zelle bzw. Durchgangsöffnung wird dabei jeweils von der Stützstruktur des Implantats berandet, die z. B. durch eine Vielzahl an miteinander verbundenen Streben gebildet sein kann. Die Streben können dabei einstückig bzw. integral miteinander verbunden sein. Insbesondere kann die Stützstruktur eine Gitterstruktur bilden. Ein solches Implantat kann z. B. durch entsprechendes Bearbeiten eines rohrförmigen (vorzugsweise metallischen) Vorformlings gebildet werden, wobei bei der Bearbeitung die Zellen bzw. Durchgangsöffnungen in das Implantat z. B. mittels Laser geschnitten werden, so dass die Stützstruktur bzw. Streben mit den Zellen (Durchgangsöffnungen) erzeugt wird. Es ist aber auch denkbar, die Stützstruktur des Implantats bzw. die Zellen/Durchgangsöffnungen des Implantats auf andere Weise zu erzeugen.

Gemäß einer Ausführungsform des erfindungsgemäßen Verfahrens ist vorgesehen, dass das Implantat im Innenraum der Hülle von einem in dem Innenraum angeordneten Rohr umgeben ist, das eine umlaufende, den Implantat sowie den Ballon umgebenden Wandung aufweist. D. h., der Implantat liegt zwischen dem (unter Druck stehenden) Ballon und dem besagten Rohr.

Weiterhin ist gemäß einer Ausführungsform des erfindungsgemäßen Verfahrens vorgesehen, dass das Rohr zumindest eine Durchgangsöffnung aufweist, die in der Wandung des Rohres ausgebildet ist.

Weiterhin ist gemäß einer Ausführungsform des erfindungsgemäßen Verfahrens vorgesehen, dass das Rohr eine Vielzahl an Durchgangsöffnungen aufweist, die in der Wandung angeordnet sind. Die einzelnen Durchgangsöffnungen können dabei äquidistant zueinander angeordnet sein.

Weiterhin ist gemäß einer Ausführungsform der Erfindung ist vorgesehen, dass das Rohr aus einem Metall gefertigt ist, insbesondere aus einem rostfreien Stahl.

Weiterhin ist gemäß einer Ausführungsform des erfindungsgemäßen Verfahrens vorgesehen, dass das Rohr einen Innendurchmesser aufweist, der einem Außendurchmesser des auf dem Ballon fixierten Implantats entspricht.

Insbesondere bildet die Vielzahl an Durchgangsöffnungen des Rohres eine Perforation des Rohres. Die mindestens eine bzw. die mehreren Durchgangsöffnungen des Rohres, die insbesondere als Düsenöffnungen zur besseren Verteilung des Unterdrucks im Innenraum der Hülle dienen, können z. B. mittels eines Lasers in das Rohr eingebracht werden.

Weiterhin ist gemäß einer Ausführungsform der Erfindung ist vorgesehen, dass die jeweilige Durchgangsöffnung des Rohres kreisförmig ausgebildet ist und einen Durchmesser aufweist, der im Bereich von 0,05 mm bis 0,1 mm liegt.

Gemäß einer Ausführungsform des erfindungsgemäßen Verfahrens können die Durchgangsöffnungen des Rohres auch als Schlitze ausgebildet sein.

Weiterhin ist gemäß einer Ausführungsform des erfindungsgemäßen Verfahrens vorgesehen, dass der Druck im Balloninnenraum im Bereich von 10 bar bis 30 bar liegt. Insbesondere liegt der Druck im Bereich von 10 bar bis 20 bar, insbesondere im Bereich von 13 bar bis 17 bar, wobei der Druck insbesondere 15 bar beträgt.

Weiterhin ist gemäß einer Ausführungsform des erfindungsgemäßen Verfahrens vorgesehen, dass der Unterdruck im Innenraum der Hülle kleiner oder gleich 0,6 bar ist, wobei der Unterdruck insbesondere im Bereich von 0,01 bar bis 0,6 bar liegt, insbesondere im Bereich von 0,1 bar bis 0,6 bar, insbesondere im Bereich von 0,3 bar bis 0,6 bar.

Weiterhin ist gemäß einer Ausführungsform des erfindungsgemäßen Verfahrens vorgesehen, dass der Ballon bei dem Erwärmen auf eine Endtemperatur erwärmt wird, die ein plastisches Verformen des Ballons bei dem besagten Druck und dem besagten Unterdruck erlaubt.

Weiterhin ist gemäß einer Ausführungsform des erfindungsgemäßen Verfahrens vorgesehen, dass die Endtemperatur in einem der folgenden Bereiche liegt:
- im Bereich von 40°C bis 150°C,
- im Bereich von 40°C bis 140°C (insbesondere für den Fall, dass der Ballon aus PA 12 besteht, siehe unten),
- im Bereich von 60°C bis 150°C (insbesondere für den Fall, dass der Ballon aus Pebax 7033 besteht, siehe unten),
- im Bereich von 50°C bis 110°C,
- im Bereich von 100°C bis 110°C, insbesondere 102°C bis 107°C (insbesondere für den Fall, dass es sich bei dem Material des Ballons um Pebax 7033 handelt und/oder für den Fall eines metallischen Implantats oder Stents, auf den insbesondere kein Medikament aufgebracht ist),
- im Bereich von 50°C bis 60°C, insbesondere 53°C bis 57°C (insbesondere für den Fall, dass es sich bei dem Material des Ballons um PA 12 handelt und/oder dass auf dem Implantat oder Stent ein Medikament aufgebracht ist, d. h. es sich um einen sogenannten Drug Eluting Stent (DES) handelt, d. h., um einen Stent, der dazu konfiguriert ist, ein Medikament abzugeben, im Gegensatz zu einem unbeschichteten Stent (BMS)).

Weiterhin ist gemäß einer Ausführungsform des erfindungsgemäßen Verfahrens vorgesehen, dass der Ballon aus einem Ballonmaterial gefertigt ist, das eine Glasübergangstemperatur aufweist, wobei die Endtemperatur größer oder gleich der Glasübergangstemperatur ist, und wobei insbesondere die Endtemperatur nicht mehr als 10%, insbesondere nicht mehr als 5%, insbesondere nicht mehr als 1% von der Glasübergangstemperatur abweicht. Die Glasübergangstemperatur kann mit einer dem Fachmann bekannten Methode bestimmt werden, wie beispielsweise thermoanalytischen Methoden (DSC, DMA, DIL, LFA).

Der Ballon wird also mit andere Worten insbesondere so erhitzt, dass er plastisch verformbar ist, wobei der Ballon sich während des durch den Druck im Balloninnenraum und den Unterdruck im Innenraum der Hülle bewirkten Tiefziehens konturtreu und dicht an den Implantat anlegt.

Gemäß einer Ausführungsform der vorliegenden Erfindung besteht der Ballon aus einem Material wie z. B. Polyamid oder Modifikationen davon, wie z. B. einem Polyether-Block - Amid (z. B. Pebax). Weiterhin kann es sich bei dem Material um ein thermoplastisches Elastomer, z. B. TPE-A, handeln. Diese teilkristallinen Kunststoffe (viele gebräuchliche Kunststoffe weisen einen kristallinen Anteil von 10% bis 80% auf) besitzen sowohl eine Glasübergangstemperatur unterhalb derer die amorphe Phase einfriert (einhergehend mit Versprödung), als auch eine Schmelztemperatur, bei der sich die kristalline Phase auflöst. Die Schmelztemperatur trennt den entropieelastischen Bereich deutlich vom Fließbereich.

Gemäß einer Ausführungsform der vorliegenden Erfindung kann der Ballon insbesondere aus einem der folgenden Materialien gefertigt sein bzw. bestehen: einem Polyamid, insbesondere PA 12, z. B. Grilamid Polyamid-12 L25; einem Polyether-Block-Amid (Peba), z. B. Pebax 3533 oder Pebax 7033; PET; PEEK; TPU.

Bei Grilamid Polyamid-12 L25 handelt es sich insbesondere um eine teilkristalline Thermoplaste mit einer Glasübergangstemperatur von 37°C sowie mit einer Schmelztemperatur von 178°C. Bei Peba bzw. PEBAX® 3533 (CAS-Nr. 77402-38-1) oder PEBAX® 7033 (CAS-Nr. 77402-38-1) handelt es sich um teilkristalline thermoplastische Elastomere (TPE), wobei z. B. PEBAX® 3533 eine Glasübergangstemperatur von -65°C und eine Schmelztemperatur von 144°C aufweist. Weiterhin kann PET (Polyethylenterephthalat) z. B. eine Glasübergangstemperatur von 70°C sowie eine Schmelztemperatur von z. B. 255°C aufweisen. Bei PEEK (Polyetheretherketon) handelt es handelt es sich insbesondere ebenfalls um eine teilkristalline Thermoplaste mit einer Glasübergangstemperatur von z. B. 143°C und einer Schmelztemperatur von z. B. 340°C. Weiterhin können im Rahmen der vorliegenden Erfindung thermoplastisches Polyurethan (TPU) als Material für den Ballon verwendet werden.

Weiterhin ist gemäß einer Ausführungsform des erfindungsgemäßen Verfahrens vorgesehen, dass der Ballon bei mit dem Druck beaufschlagten Balloninnenraum und bei dem im Innenraum der Hülle angelegten Unterdruck über einen Zeitraum von zumindest 10 s, inbesondere zumindest 20 s, insbesondere zumindest 30 s, insbesondere zumindest 40 s, insbesondere zumindest 50 s, insbesondere zumindest 60s, der besagten Endtemperatur ausgesetzt wird.

Gemäß einer Ausführungsform des Verfahren ist vorgesehen, dass der Ballon bei mit dem Druck beaufschlagten Balloninnenraum und bei dem im Innenraum der Hülle angelegten Unterdruck über einen Zeitraum von zumindest 10 s bis 100 s, insbesondere 20 s bis 80 s, insbesondere 30 s bis 60 s, insbesondere 20 s bis 40 s, insbesondere 25 s bis 35 s, insbesondere 50 s bis 70 s, insbesondere 55 s bis 65 s, der besagten Endtemperatur ausgesetzt wird.

Die Zeiträume 20 s bis 40 s bzw. 25 s bis 35 s, werden insbesondere verwendet, wenn es sich bei dem Implantat um einen Stent handelt, der dazu konfiguriert ist ein Medikament abzugeben (DES). Hierbei kann der Ballon z. B. aus PA 12 oder einem anderen Polyamid bestehen.

Die Zeiträume 50 s bis 70 s bzw. 55 s bis 65 s, werden insbesondere verwendet, wenn es sich bei dem besagten Implantat um einen rein metallischen Stent (BMS) handelt. Hierbei kann der Ballon z. B. aus PEBAX® 7033 oder einem sonstigen TPE, insbesondere TPE-A, bestehen.

Im Hinblick auf die verschiedenen Stent-Konfigurationen (DES oder BMS) kann die vorliegende Erfindung insbesondere mit den folgenden beispielhaften Parametern durchgeführt werden:

| Stent | Ballonmaterial | Endtemperatur °(C) | Zeitraum bzw. Prozessdauer (s) | Druck im Balloninnenraum (bar) |
|---|---|---|---|---|
| BMS | PEBAX® 7033 | 105+/- 3 | 60 | 15 +/- 0.5 |
| DES | PA 12 | 55+/- 2 | 30 | 15 +/- 0.5 |

Weiterhin ist gemäß einer Ausführungsform des erfindungsgemäßen Verfahrens vorgesehen, dass der Ballon mittels Joulscher Wärme, insbesondere mittels eines elektrischen Leiters, erwärmt wird. Weiterhin befindet sich der Ballon beim Erwärmen vorzugsweise im Innenraum der Hülle und ist dabei insbesondere von dem besagten Rohr umgeben. Der besagte elektrische Leiter kann an der Hülle angeordnet bzw. in diese eingebettet sein. Der Ballon zusammen mit dem darauf gecrimpten Implantat kann jedoch auch außerhalb der Hülle erwärmt werden (siehe oben).

Weiterhin ist gemäß einer Ausführungsform des erfindungsgemäßen Verfahrens vorgesehen, dass der Balloninnenraum mit dem Druck beaufschlagt wird bevor der Ballon erwärmt wird, und/oder dass der Unterdruck an den Innenraum der Hülle angelegt wird, bevor der Ballon erwärmt wird. Dies gilt insbesondere für den Fall, bei dem der Ballon im Innenraum der Hülle erwärmt wird.

Es ist jedoch auch denkbar, den Unterdruck zeitgleich mit dem Erwärmen oder erst nach dem Erwärmen des Ballons auf die Endtemperatur im Innenraum anzulegen.

Ferner ist es auch denkbar, den Balloninnenraum zeitgleich mit dem Erwärmen des Ballons oder erst nach dem Erwärmen des Ballons auf die Endtemperatur mit dem Druck zu beaufschlagen.

Gemäß einem weiteren Aspekt der vorliegenden Erfindung wird eine Anordnung offenbart, aufweisend ein Implantat, insbesondere einen Stent, das auf einen Ballon gecrimpt ist, wobei das Implantat mittels eines erfindungsgemäßen Verfahrens an dem Ballon fixiert ist.

Gemäß einem weiteren Aspekt der vorliegenden Erfindung wird eine Vorrichtung zum Fixieren eines Implantats, insbesondere eines Stents, an einem Ballon offenbart, wobei die Vorrichtung insbesondere bei dem erfindungsgemäßen Verfahren verwendet wird.

Die erfindungsgemäße Vorrichtung weist zumindest auf:
- eine Hülle, die einen Innenraum zur Aufnahme eines auf einen Ballon gecrimpten Implantats aufweist,
- eine mit dem Innenraum in Strömungsverbindung bringbare Pumpe, die dazu konfiguriert ist, im Innenraum der Hülle einen Unterdruck zu erzeugen,
- eine Einrichtung, die mit einem Balloninnenraum des Ballons in Strömungsverbindung brinbar ist und zum Beaufschlagen des Balloninnenraumes des Ballons mit einem Druck konfiguriert ist, und
- eine Heizeinrichtung, die dazu konfiguriert ist, den Ballon zu erwärmen, insbesondere wenn der Ballon im Innenraum der Hülle angeordnet ist (oder wenn der Ballon außerhalb des Innenraumes der Hülle angeordnet ist bzw. bevor der Ballon in dem Innenraum der Hülle angeordnet wird).

Gemäß einer Ausführungsform der erfindungsgemäßen Vorrichtung ist vorgesehen, dass die Vorrichtung ein Rohr aufweist, das dazu konfiguriert ist, den Implantat zu umgeben, wenn der Implantat im Innenraum der Hülle angeordnet ist, so dass der Implantat zwischen dem Ballon und dem Rohr angeordnet ist.

Weiterhin ist gemäß einer Ausführungsform der erfindungsgemäßen Vorrichtung vorgesehen, dass das Rohr zumindest eine Durchgangsöffnung oder eine Vielzahl an Durchgangsöffnungen aufweist, wobei die jeweilige Durchgangsöffnung in einer umlaufenden Wandung des Rohres ausgebildet ist. Hinsichtlich weiterer Merkmale des Rohres der Vorrichtung wird auf die oben bereits beschriebenen Merkmale des Rohres verwiesen.

Weiterhin ist gemäß einer Ausführungsform der erfindungsgemäßen Vorrichtung ist vorgesehen, dass die Hülle der Vorrichtung durch eine starre Form gebildet ist, wobei die Hülle eine hermetisch verschließbare Öffnung aufweist, über die der Ballon samt Implantat im Innenraum der Hülle anordenbar ist, und wobei eine mit dem Balloninnenraum kommunizierende Leitung über eine hermetisch abdichtbare Durchführung der Hülle aus der Hülle herausführbar ist. Die Einrichtung zur Erzeugung des Drucks im Balloninnenraum ist vorzugsweise dazu konfiguriert, mit dieser Leitung in Strömungsverbindung gebracht zu werden, um den Balloninnenraum mit dem Druck zu beaufschlagen. Bei der Leitung kann es sich um ein Lumen eines mit dem Ballon verbundenen Katheters handeln.

Weitere Merkmale und Ausführungsformen der vorliegenden Erfindung sollen nachfolgend im Zusammenhang mit den Figuren beschrieben werden. Es zeigen:
- Fig. 1: eine schematische Darstellung einer Vorrichtung die zur Durchführung des erfindungsgemäßen Verfahrens verwendbar ist;
- Fig. 2: einen Schnitt durch einen Stent, der auf einen Ballon gecrimpt ist, und in der Vorrichtung gemäß Figur 1 angeordnet ist.

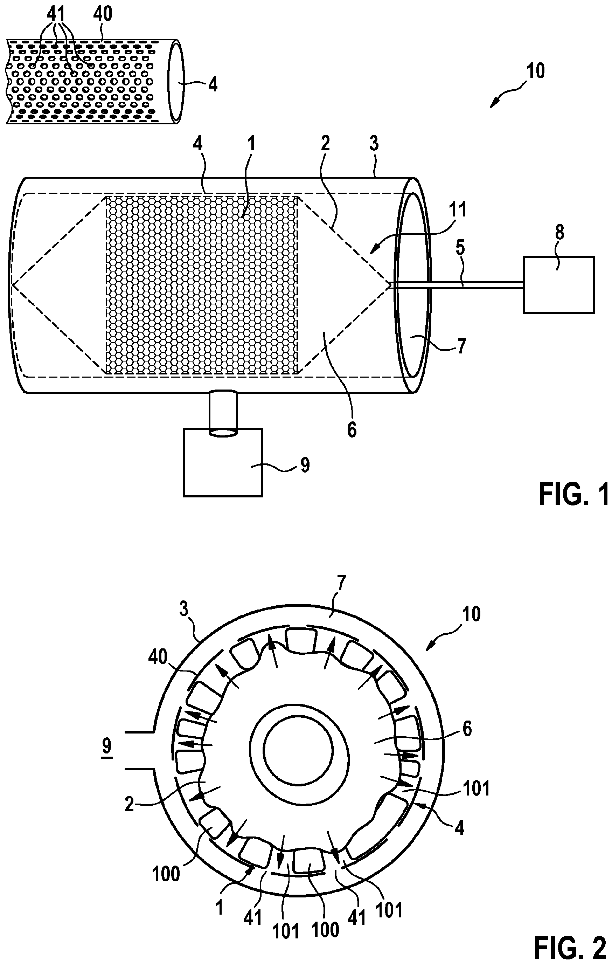

Figur 1 zeigt im Zusammenhang mit Figur 2 eine Vorrichtung 10, die zur Durchführung des erfindungsgemäßen Verfahrens konfiguriert ist. Hierbei wird das erwärmte Ballonmaterial unter dem Druck im Balloninnenraum 6 mit Hilfe eines von außen angelegten Unterdrucks (insbesondere Vakuum) in Durchgangsöffnungen 101 des Stents 1 tiefgezogen, die jeweils von einer Stützstruktur 100 des Stents 1 umrandet sind (vgl. auch Fig. 2).

Die Vorrichtung 10 weist eine mittels einer Heizeinrichtung 20 beheizbaren Form bzw. Hülle 3 auf. Die Hülle 3 kann dabei mittels elektrischer Heizelemente oder induktiv erwärmt werden. Alternativ oder zusätzlich kann der Ballon 2 jedoch auch außerhalb der Hülle 3 mittels einer separaten Heizvorrichtung 20 auf die notwendige Endtemperatur erwärmt werden und dann im Innenraum 7 der Hülle 3 angeordnet werden.

Die Hülle 3 ist weiterhin an eine Pumpe 9 zum Erzeugen eines Unterdrucks im Innenraum 7 der Hülle 3 angeschlossen. Im Innenraum 7 der Hülle 3 ist vorzugsweise ein perforiertes Rohr 4 angeordnet, wobei insbesondere der Innendurchmesser des Rohrs 4 dem Enddurchmesser des auf den Ballon 2 gecrimpten Stents 1 entspricht. Gemäß einer Ausführungsform wird die Perforation des Rohres 4 durch eine Vielzahl an Durchgangsöffnungen 41 bzw. Düsenbohrungen 41 des Rohres 4 gebildet, die z. B. mit Hilfe eines Lasers in die Wandung 40 des Rohres 4 geschnitten werden. Der Durchmesser dieser Löcher 41 kann z. B. bei 0,05 mm bis 0,1 mm liegen. Als Alternative kann das Rohr 4 mit Schlitzen statt Löchern verwendet werden. Das Material des Rohrs 4 kann aus rostfreiem Stahl bestehen. Zum Beaufschlagen des Balloninnenraumes 6 mit einem Druck ist eine mit dem Ballon 2 in Strömungsverbindung stehende Leitung 5 aus der Hülle 3 bzw. der Form 3 herausgeführt, wobei eine Einrichtung 8 zur Erzeugung des Drucks mit dieser Leitung 5 verbunden wird. Bei der Leitung kann es sich um Lumen eines mit dem Ballon 2 verbundenen Katheters handeln.

Der Stent 1 ist zwischen dem gefalteten Ballon 2 und dem perforierten Rohr 4 angeordnet (vgl. insbesondere Fig. 2). Zum Tiefziehen des erwärmten Ballons 2 wird nun der Balloninnenraum 6 mit dem Druck beaufschlagt, wohingegen der Innenraum 7 der Hülle 3 evakuiert wird, so dass dort der besagte Unterdruck anliegt. Der Druck im Ballonrinnenraum 6 beträgt beispielsweise 15 bar und der Unterdruck im Innenraum 7 der Hülle 3 liegt beispielsweise im Bereich von circa 0,3 bar bis 0,6 bar. Der Ballon 2 mit dem darauf gecrimptem Stent 1 kann zum Tiefziehen (bedingt durch den besagten Druck und den besagten Unterdruck) mittels der Heizeinrichtung (z. B. in Form einer Heizspirale) 20 auf eine Endtemperatur (z. B. oberhalb der Glasübergangstemperatur des Ballonmaterials und unterhalb der Schmelztemperatur) erhitzt werden, damit er plastisch verformbar ist. Diese Endtemperatur kann z. B. bei rein metallischen Stents (BMS) bei 105°C und für sogenannte DES (Drug Eluting Stents) bei beispielsweise 55°C liegen. Hierzu wird auch auf die oben beschriebenen Ausführungsformen und Beispiele verwiesen. Während des Tiefziehens, das insbesondere über einen vordefinierten Zeitraum erfolgt (z. B. 30 s bei DES oder 60 s bei BMS, siehe auch oben) legt sich der Ballon 2 nun konturtreu und dicht an den Stent 1 an, wobei sich, wie in der Figur 2 anhand von Pfeilen angedeutet, einzelne Materialbereiche des Stents 1 plastisch verformen und dabei in die Zellen 101 bzw. Durchgangsöffnungen 101 des Stents 1 eindringen und daher dort jeweils einen Formschluss mit der Stützstruktur 100 des Stents 1 erzielen.

Durch die erfindungsgemäße Lösung wird somit im Ergebnis eine optimale Formschlussverbindung zwischen Stent 1 und Ballon 2 erzeugt, die die Stenthaltekraft erhöht. Hierdurch verringert sich in vorteilhafter Weise das Risiko eines Verschiebens des Stents (vgl. "Stent Displacement" gemäß ASTM F2394-07) sowie das Risiko eines Lösens des Stents vom Ballon (vgl. "Stent Dislodgment" gemäß ASTM F2394-07).

## Patentansprüche

1. Verfahren zum Fixieren eines Implantats (1) auf einen Ballon (2), wobei ein, bevorzugt erwärmter, Ballon (2), auf den ein Implantat (1) gecrimpt ist, das eine Stützstruktur (100) mit einer Vielzahl an Durchgangsöffnungen (101) aufweist, in einem Innenraum (7) einer Hülle (3) bereitgestellt wird, wobei im Innenraum (7) der Hülle (3) ein Unterdruck angelegt wird und ein Balloninnenraum (6) des Ballons (2) mit einem Druck beaufschlagt wird, so dass der Ballon (2) in die Durchgangsöffnungen (101) des Implantats (1) tiefgezogen wird, wobei ein Formschluss zwischen dem Ballon (2) und der Stützstruktur (100) des Implantats (1) erzeugt wird.

2. Verfahren nach Anspruch 1, wobei das Bereitstellen des erwärmten Ballons (2) im Innenraum (7) der Hülle (3) erfolgt, indem der Ballon (2) außerhalb des Innenraumes (7) der Hülle (3) erwärmt wird und sodann der erwärmte Ballon (2) samt Implantat (1) im Innenraum (7) der Hülle (3) angeordnet wird, oder indem der Ballon (2) samt Implantat (1) im Innenraum (7) der Hülle (3) angeordnet wird und im Innenraum (7) der Hülle (3) erwärmt wird.

3. Verfahren nach Anspruch 1 oder 2, wobei das Implantat (1) im Innenraum (7) der Hülle (3) von einem in dem Innenraum (7) angeordneten Rohr (4) umgeben ist, das eine umlaufende, den Implantat (1) sowie den Ballon (2) umgebende Wandung (40) aufweist.

4. Verfahren nach Anspruch 3, wobei das Rohr (4) zumindest eine Durchgangsöffnung (41) aufweist, die in der Wandung (40) des Rohres (4) ausgebildet ist.

5. Verfahren nach Anspruch 3, wobei das Rohr (4) eine Vielzahl an in der Wandung (40) angeordneten Durchgangsöffnungen (41) aufweist.

6. Verfahren nach einem der vorhergehenden Ansprüche, wobei der Druck im Balloninnenraum (6) im Bereich von 10 bar bis 30 bar liegt, insbesondere im Bereich von 14,5 bar bis 15,5 bar.

7. Verfahren nach einem der vorhergehenden Ansprüche, wobei der Unterdruck im Innenraum (7) der Hülle (3) kleiner oder gleich 0,6 bar ist, wobei der Unterdruck insbesondere im Bereich von 0,01 bar bis 0,6 bar liegt, insbesondere im Bereich von 0,1 bar bis 0,6 bar, insbesondere im Bereich von 0,3 bar bis 0,6 bar.

8. Verfahren nach einem der vorhergehenden Ansprüche, wobei der Ballon (2) bei dem Erwärmen des Ballons (2) auf eine Endtemperatur erwärmt wird.

9. Verfahren nach Anspruch 8, wobei die Endtemperatur in einem der folgenden Bereiche liegt:
- im Bereich von 40°C bis 150°C,
- im Bereich von 40°C bis 140°C [insbesondere für den Fall, dass der Ballon aus PA 12 besteht],
- im Bereich von 60°C bis 150°C [insbesondere für den Fall, dass der Ballon aus PEBAX® 7033 besteht],
- im Bereich von 50°C bis 110°C,
- im Bereich von 100°C bis 110°C, insbesondere 102°C bis 107°C [insbesondere für den Fall, dass es sich bei dem Material des Ballons um PEBAX® 7033 handelt]
- im Bereich von 50°C bis 60°C, insbesondere 53°C bis 57°C [insbesondere für den Fall, dass es sich bei dem Material des Ballons um PA 12 handelt]

10. Verfahren nach Anspruch 8 oder 9, wobei der Ballon (2) aus einem Ballonmaterial gefertigt ist, das eine Glasübergangstemperatur aufweist, wobei die Endtemperatur größer oder gleich der Glasübergangstemperatur ist, und wobei insbesondere die Endtemperatur nicht mehr als 10%, insbesondere nicht mehr als 5%, insbesondere nicht mehr als 1% von der Glasübergangstemperatur abweicht.

11. Verfahren nach einem der Ansprüche 8 bis 10, wobei der Ballon (2) bei mit dem Druck beaufschlagten Balloninnenraum (6) und bei dem im Innenraum (7) der Hülle (3) angelegten Unterdruck über einen Zeitraum von 10 s bis 100 s, insbesondere 20 s bis 80 s, insbesondere 30 s bis 60 s, insbesondere 20 s bis 40 s, insbesondere 25 s bis 35 s, insbesondere 50 s bis 70 s, insbesondere 55 s bis 65 s, der besagten Endtemperatur ausgesetzt wird.

12. Verfahren nach einem der vorhergehenden Ansprüche, wobei der Balloninnenraum (6) mit dem Druck beaufschlagt wird bevor der Ballon (2) erwärmt wird, und/oder dass der Unterdruck an den Innenraum (7) der Hülle (3) angelegt wird, bevor der Ballon (2) erwärmt wird.

13. Anordnung (11), aufweisend einen Implantat (1), der auf einen Ballon (2) gecrimpt ist, wobei der Implantat (1) mittels des Verfahrens nach einem der vorhergehenden Ansprüche an dem Ballon (2) fixiert ist.

14. Vorrichtung (10) zum Fixieren eines Implantats (1) an einem Ballon (2), mit:
- einer Hülle (3), die einen Innenraum (7) zur Aufnahme eines auf einen Ballon (2) gecrimpten Implantats (1) aufweist,
- einer mit dem Innenraum (7) in Strömungsverbindung bringbaren Pumpe (9), die dazu konfiguriert ist, im Innenraum (7) der Hülle (3) einen Unterdruck zu erzeugen,
- einer Einrichtung (8), die mit einem Balloninnenraum (6) des Ballons (2) in Strömungsverbindung bringbar ist und zum Beaufschlagen des Balloninnenraumes (6) des Ballons (2) mit einem Druck konfiguriert ist, und
- einer Heizeinrichtung (20), die dazu konfiguriert ist, den Ballon (2) zu erwärmen.

15. Vorrichtung nach Anspruch 14, wobei die Vorrichtung ein Rohr (4) aufweist, das dazu konfiguriert ist, den Implantat (1) zu umgeben, wenn der Implantat (1) im Innenraum (7) der Hülle (3) angeordnet ist, so dass der Implantat (1) zwischen dem Ballon (2) und dem Rohr (4) angeordnet ist.

16. Vorrichtung nach Anspruch 15, wobei das Rohr (4) zumindest eine in einer umlaufenden Wandung (40) des Rohres (4) angeordnete Durchgangsöffnung (41) oder eine Vielzahl an in einer umlaufenden Wandung (40) des Rohres (4) angeordnete Durchgangsöffnungen (41) aufweist.
